# FASCICULE DE BREVET EUROPEEN

(11) **EP 2 229 943 B1**
(45) Date de publication et mention de la délivrance du brevet: **04.07.2012**
(21) Numéro de dépôt: 10305260.1
(22) Date de dépôt: 16.03.2010
(51) Int. Cl.: A61K 31/357, A61K 31/36, A61K 45/06, A61P 25/02

(54) **Composé pour son utilisation dans le traitement des neuropathies périphériques**
Verbindungen zur Behandlung der peripheren Neuropathie
Compounds for use in the treatment of peripheral neuropathy

(30) Priorité: 19.03.2009 FR 0951766
(43) Date de publication de la demande: 22.09.2010
(73) Titulaire: Biocodex, 94250 Gentilly (FR)
(72) Inventeur: Le Guern, Marie-Emmanuelle, 60200 Compiegne (FR); Verleye, Marc, 60190 Remy (FR); Gillardin, Jean-Marie, 60680 Jonquieres (FR); Hublot, Bernard, 60200 Compiegne (FR)
(74) Mandataire: Vial, Lionel

(56) Documents cités:
- WO-A2-2009/132119
- STOJKOVIC, T; DONZÉ, C: "Neuropathies peripheriques. Le point sur les traitements symptomatiques" NEUROLOGIES, vol. 3, septembre 2001 (2001-09), pages 291-301, XP007909522
- CHIRON CATHERINE: "Stiripentol." EXPERT OPINION ON INVESTIGATIONAL DRUGS JUL 2005, vol. 14, no. 7, juillet 2005 (2005-07), pages 905-911, XP002541844 ISSN: 1744-7658
- APFEL STUART C: "Neurotrophic factors and diabetic peripheral neuropathy" EUROPEAN NEUROLOGY, vol. 41, no. SUPPL. 1, février 1999 (1999-02), pages 27-34, XP008110124 ISSN: 0014-3022
- Oudard S: "Progrès en urologie", vol. 12, pages 19-30,

## Description

La présente invention concerne un composé pour son utilisation dans le traitement des neuropathies périphériques.

Les neuropathies périphériques, également connues sous le nom de névrites périphériques, concernent l'ensemble des maladies des nerfs périphériques. On distingue les neuropathies périphériques asymétriques, qui regroupent les mononeuropathies uniques ou multiples, et les neuropathies périphériques symétriques ou polyneuropathies.

Comme cela est rappelé dans la synthèse des recommandations professionnelles relative à la prise en charge diagnostique des neuropathies périphériques, émise par la Haute Autorité de Santé (HAS, France) en Mai 2007, les neuropathies périphériques peuvent se révéler par :
- des symptômes sensitifs : paresthésie, dysesthésie, hypoesthésie, douleur, troubles de l'équilibre, symptômes subjectifs distaux ;
- des symptômes moteurs : faiblesse, en particulier des loges antéro-externes des jambes, une faiblesse proximale ou diffuse, des crampes musculaires au repos ou des fasciculations, ou
- des symptômes neurovégétatifs : malaises orthostatiques ou post-prandiaux, troubles de la sudation, troubles mictionnels, troubles de l'érection et de l'éjaculation, diarrhée motrice, sensation de plénitude gastrique, symptômes trophiques, apparition d'une hyperkératose puis d'une ulcération indolore aux points d'appui de la plante des pieds.

Chez l'enfant, les circonstances de révélation peuvent être particulières : troubles de l'écriture, hypotonie aréflexique (chez le nourrisson), retard d'acquisition motrice et pieds plats valgus (chez l'enfant de moins de 4 ans).

En général, le tableau d'une neuropathie périphérique est sensitivo-moteur et symétrique.

Les étiologies les plus fréquentes des neuropathies périphériques regroupent, notamment, le diabète en cours de traitement, une consommation régulière et excessive d'alcool, ainsi qu'une insuffisance rénale chronique. Il peut également s'agir de neuropathies infectieuses (notamment dues à un zona), de neuropathies radiques, de neuropathies liées à un processus inflammatoire, de neuropathies faisant suite à des lésions post-traumatiques ou post-chirurgicales (post-sciatiques, par exemple), de neuropathies liées à d'éventuels antécédents familiaux de neuropathie, et de neuropathies liées à la prise de certains médicaments, notamment des classes suivantes : antimitotiques, antibiotiques, antiviraux, antiarythmiques, antirhumatismaux, immunosuppresseurs, antipsychotiques, antiépileptiques, antilépreux ou antituberculeux.

S'agissant plus particulièrement des neuropathies périphériques induites par la prise de certains médicaments, le cas se produit notamment lors de chimiothérapies anticancéreuses. En effet, de nombreux médicaments, principalement à activité cytostatique ou anti-mitotique, utilisés dans ces thérapies induisent une neuropathie périphérique se manifestant en général par des symptômes sensitifs.

Ainsi, le cisplatine, utilisé dans le traitement de différents cancers, a pour principal effet secondaire d'induire l'apparition de neuropathies périphériques sensitives, notamment par la perte de sensibilité des extrémités distales, associées à une dégénérescence axonale des neurones sensitifs (Thompson et al. (1984) Cancer 54:1269-1275).

La principale réponse consiste à diminue, les doses administrées de ces médicaments ou bien à interrompre le traitement, ce qui diminue d'autant leur efficacité thérapeutique.

Il est donc nécessaire de disposer de composés neuroprotecteurs susceptibles de prévenir ou de traiter les neuropathies périphériques, notamment liées à la prise de médicaments.

A ce titre, le *Nerve Growth Factor* (NGF, Facteur de Croissance des Nerfs) est le composé visant le traitement des neuropathies périphériques sur lequel le plus de données sont disponibles. Il a ainsi été montré *in vitro* que le NGF s'opposait à la réduction liée au cisplatine de la croissance des neurites de ganglions rachidiens dorsaux de rats (Konings et al. (1994) Brain Res. 640:195-204), et *in vivo,* chez la souris, que le NGF favorisait la guérison d'une neuropathie périphérique induite par le cisplatine (Aloe et al. (2000) Auton Neurosci. 86:84-93). Toutefois, les différents essais cliniques menés chez l'homme avec le NGF n'ont pas été concluant, principalement du fait des effets secondaires liés à son utilisation, conduisant à envisager des modes d'administration par transfert de gène (Chattopadhyay et al. (2004) Brain 127:929-939), dont les difficultés de mise en oeuvre sont bien connues.

Il reste donc à trouver un composé alternatif au NGF pour le traitement des neuropathies périphériques qui soit susceptible d'être administré de manière aisée chez l'homme.

Le stiripentol (Diacomit), ou 4,4-diméthyl-1-[(3,4-méthylènedioxy)-phényl]-1-pentène-3-ol, est un anti-épileptique indiqué dans l'épilepsie myoclonique sévère du nourrisson, en addition à l'association valproate de sodium et clobazam, lorsque celle-ci s'avère insuffisante pour contrôler les crises (Chiron et al. (2000) Lancet 356:1638-1642).

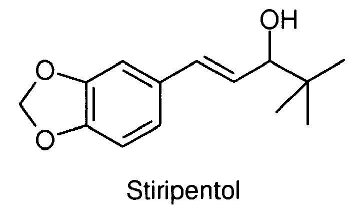

Parmi ses principaux effets, le stiripentol inhibe la recapture de l'acide gamma amino-butyrique (GABA) et est par ailleurs un inhibiteur de plusieurs isoenzymes du cytochrome P450, notamment CYP1A2 et CYP3A4 (Tran et al. (1997) Clin. Pharmacol. Ther. 62:490-504).

### Résumé de l'invention

La présente invention découle de la mise en évidence inattendue, par les inventeurs, que le stiripentol présentait un effet neuroprotecteur équivalent à celui du NGF dans un modèle *in vitro* de neuropathie périphérique.

Ainsi, la présente invention concerne un composé de formule (I) suivante, 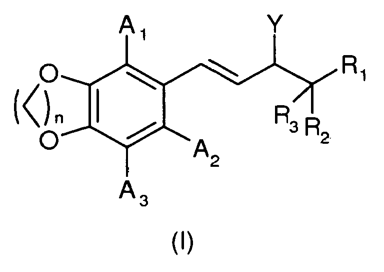 dans laquelle :
- n représente 1 ou 2,
- A₁, A₂ et A₃, identiques où différents, représentent un atome d'hydrogène, un atome d'halogène ou un groupe alkyle linéaire ou ramifié de 1 à 4 atomes de carbone,
- R₁, R₂ et R₃ représentent indépendamment un atome d'hydrogène ou un groupe alkyle linéaire ou ramifié de 1 à 4 atomes de carbones, et
- Y représente -OH, =O ou -SH ;
ou un sel pharmaceutiquement acceptable de celui-ci,
pour son utilisation dans le traitement des neuropathies périphériques.

La présente invention concerne également la prévention ou le traitement des neuropathies périphériques chez un individu, dans lesquels on administre à l'individu une quantité prophylactiquement ou thérapeutiquement efficace d'un composé de formule (I) tel que défini ci-dessus, ou d'un sel pharmaceutiquement acceptable de celui-ci.

Dans un mode de réalisation particulier du composé ou de la prévention ou du traitement définis ci-dessus, le composé de formule (I), ou le sel pharmaceutiquement acceptable de celui-ci, est combiné à au moins un composé additionnel destiné à la prévention ou au traitement des neuropathies périphériques et/ou à au moins un composé additionnel cytostatique ou anti-mitotique tel que défini dans les revendications.

La présente invention concerne également une composition pharmaceutique, comprenant à titre de substances actives, au moins un composé de formule (I) tel que défini ci-dessus, ou un sel pharmaceutiquement acceptable de celui-ci, et au moins un composé additionnel destiné à la prévention ou au traitement des neuropathies périphériques et/ou au moins un composé additionnel cytostatique ou anti-mitotique tel que défini dans les revendications, éventuellement en association avec un véhicule pharmaceutiquement acceptable.

La présente invention concerne également des produits contenant :
- au moins un composé de formule (I) tel que défini ci-dessus, ou un sel pharmaceutiquement acceptable de celui-ci, et
- au moins un composé additionnel destiné à la prévention ou au traitement des neuropathies périphériques, et/ou
- au moins un composé additionnel cytostatique ou anti-mitotique,
comme produit de combinaison pour une utilisation simultanée, séparée ou étalée dans le temps pour la prévention ou le traitement des neuropathies périphériques.

### Description détaillée de l'invention

### Composés de formule (I)

De préférence la formule (I) ci-dessus est représentée par la formule (II) suivante : 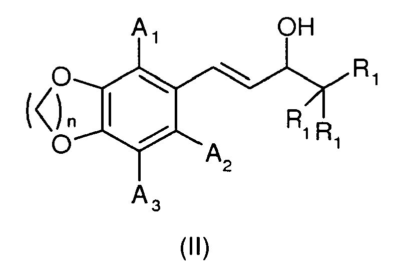 dans laquelle n, A₁, A₂, A₃ et R₁ sont tels que définis ci-dessus.

Plus préférablement, la formule (I) ou (II) ci-dessus est représentée par la formule (III) suivante :

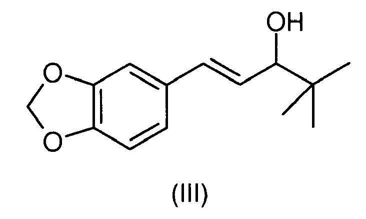

Le composé de formule (III) est le stiripentol ou 4-diméthyl-1-[(3,4-méthylènedioxy)-phényl]-1-penten-3-ol.

Comme cela apparaîtra clairement à l'homme du métier, les formules (I), (II), et (III) définies ci-dessus représentent les différents stéréoisomères compris dans ces formules ou leurs mélanges, en particulier leurs mélanges racémiques.

Ainsi, le composé de formule (III) peut être un composé de formule (IIIa), un composé de formule (IIIb), ou un mélange du composé de formule (IIIa) et du composé de formule (IIIb), en particulier le mélange racémique de ces derniers.

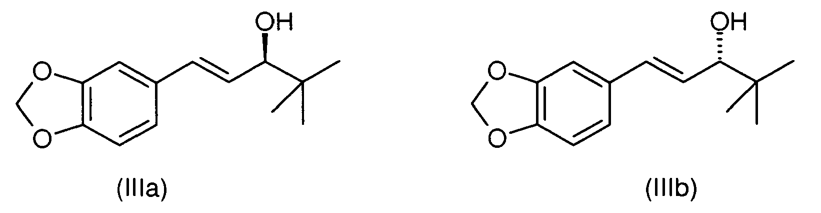

Parmi les groupes alkyles préférés selon l'invention, on peut notamment citer les groupes méthyle, éthyle, n-propyle, isopropyle, n-butyle, s-butyle et t-butyle. Les atomes de chlore, d'iode, de brome ou de fluor sont des atomes d'halogène préférés selon l'invention.

Le brevet FR 2 173 691 décrit la synthèse du stiripentol, en particulier à partir de la (méthylènedioxy-3,4-phényl)-1-diméthyl-4,4-pentèn-1-on-3. L'homme du métier pourra également aisément synthétiser les autres composés de formule (I) à partir de cet enseignement.

### Utilisation thérapeutique

Les « neuropathies périphériques » sont bien connues de l'homme du métier et sont notamment définies dans la synthèse des recommandations professionnelles relative à la prise en charge diagnostique des neuropathies périphériques, émise par la Haute Autorité de Santé (HAS, France) en Mai 2007 et dans l'article (Bouhassira (2008) Presse Med. 37:311-314) ainsi que dans l'article (Bouhassira et al. (2008) Pain 136:380-7).

Plus particulièrement, les neuropathies selon l'invention peuvent être des neuropathies périphériques asymétriques ou symétriques. De préférence, les neuropathies périphériques selon l'invention sont symétriques.

Par ailleurs, les neuropathies périphériques selon l'invention sont de préférence associées à, ou se manifestent par :
- des symptômes sensitifs : tels qu'une paresthésie, une dysesthésie, une hypoesthésie, une douleur, des troubles de l'équilibre, ou des symptômes subjectifs distaux ; ou
- des symptômes moteurs : tels qu'une faiblesse, en particulier des loges antéro-externes des jambes, une faiblesse proximale ou diffuse, des crampes musculaires au repos ou des fasciculations, ou
- des symptômes neurovégétatifs : tels que des malaises orthostatiques ou post-prandiaux, des troubles de la sudation, des troubles mictionnels, des troubles de l'érection et/ou de l'éjaculation, une diarrhée motrice, une sensation de plénitude gastrique, des symptômes trophiques, l'apparition d'une hyperkératose puis d'une ulcération indolore aux points d'appui de la plante des pieds.

Plus préférablement, les neuropathies périphériques selon l'invention sont associées à ou se manifestent par des symptômes sensitivo-moteurs, en particulier par des symptômes sensitifs, et plus particulièrement par une douleur.

Les neuropathies périphériques selon l'invention peuvent notamment être liées à, provenir de, ou, être induites par un diabète en cours de traitement, une consommation régulière et excessive d'alcool, une insuffisance rénale chronique, une infection (neuropathie infectieuse), telle que le zona, une irradiation (neuropathie radique), un processus inflammatoire, de lésions post-traumatiques ou post-chirurgicales, par exemple post-sciatiques, d'éventuels antécédents familiaux de neuropathie, et de la prise de certains médicaments, en particulier des classes suivantes : antimitotiques, antibiotiques, antiviraux, antiarythmiques, antirhumatismaux, immunosuppresseurs, antipsychotiques, antiépileptiques, antilépreux et antituberculeux. Toutefois, il est préféré, dans le cadre de la présente invention, que les neuropathies périphériques soient liées à, induites par, ou proviennent de la prise d'un médicament, notamment cytostatique ou anti-mitotique.

Les médicaments ou composés cytostatiques ou anti-mitotiques sont bien connus de l'homme du métier. Il s'agit de médicaments ou de composés empêchant ou limitant la multiplication ou la division cellulaire. Ces médicaments ou composés sont notamment indiqués pour le traitement des maladies dans lesquelles une multiplication cellulaire anormale se produit, telles que les cancers et les maladies prolifératives.

De préférence, les médicaments ou composés cytostatiques ou antimitotiques selon l'invention sont sélectionnés dans le groupe constitué des dérivés du platine, tels que le cisplatine, le satraplatine, le carboplatine, et l'oxaliplatine, de la vincristine, de la vinblastine, de la doxorubicine et des taxoïdes.

### Posologie et administration

De préférence, le composé de formule (I) tel que défini ci-dessus, ou le sel pharmaceutiquement acceptable de celui-ci, est administré à une dose unitaire de 5 mg/kg à 100 mg/kg. Par ailleurs, le composé de formule (I) tel que défini ci-dessus, ou le sel pharmaceutiquement acceptable de celui-ci, est de préférence administré avec un régime de dosage de 10 mg/kg/j à 200 mg/kg/j.

De préférence également, le composé de formule (I) tel que défini ci-dessus, ou le sel pharmaceutiquement acceptable de celui-ci, est administré sous une forme convenant pour une administration par voie orale ou rectale. Ainsi, le composé de formule (I) tel que défini ci-dessus, ou le sel pharmaceutiquement acceptable de celui-ci, est de préférence présenté sous la forme d'une poudre, de sachets, de comprimés, de gélules ou de suppositoires.

### Composé additionnel

Comme on l'entend ici, des composés ou des produits sont « combinés » ou en « combinaison » lorsqu'ils sont associés de façon qu'ils puissent interagir, ou que leurs effets se recouvrent temporellement, chez l'individu auquel ils sont administrés. Ainsi, les composés ou les produits peuvent être administrés ensemble, au sein d'une même composition pharmaceutique, ou bien séparément, c'est-à-dire sous des formes galéniques différentes et/ou par des voies d'administration distinctes et/ou à des temps ou des durées d'administration distinctes.

Lorsque le composé de formule (I) tel que défini ci-dessus, ou le sel pharmaceutiquement acceptable de celui-ci, est combiné à au moins un composé additionnel destiné à la prévention ou au traitement des neuropathies périphériques, leur administration est de préférence telle que leurs effets thérapeutiques se cumulent, de manière additive ou synergique.

Des composés additionnels destinés à la prévention ou au traitement des neuropathies périphériques selon l'invention sont bien connus de l'homme du métier et sont notamment décrits dans Stojkovic & Donzé (2001) Neurologies 3:291-301 et dans la conférence de consensus et recommandations de la Canadian Pain Society (2007) Pain Res Manag. 12:13-21. Il s'agit généralement de composés neuroprotecteurs et/ou analgésiques. De préférence, dans le cadre de la présente invention, le composé additionnel destiné à la prévention ou au traitement des neuropathies périphériques est sélectionné dans le groupe constitué du NGF, du BDNF, du CNTF, de l'IGF-I, du NT-3 et de la L-carnitine.

Le BDNF (*brain-derived neurotrophic factor,* facteur neurotrophique dérivé du cerveau), le CNTF (*ciliary neurotrophic factor,* facteur neurotrophique ciliaire), l'IGF-I (*Insulin-like growth factor-I,* facteur de croissance analogue à l'insuline-I), et NT-3 (neurotrophine-3) sont notamment décrits dans Apfel & Kessler (1995) Bailliere's Clin. Neurol. 4:593-606. La L-carnitine est notamment décrite dans Uzun et al. (2005) Electromyogr. Clin. Neurophysiol. 45:343-51.

Lorsque le composé de formule (I) tel que défini ci-dessus, ou le sel pharmaceutiquement acceptable de celui-ci, est combiné à au moins un composé additionnel cytostatique ou anti-mitotique tel que défini ci-dessus, leur administration est de préférence telle que les effets du composé de formule (I) tel que défini ci-dessus, ou du sel pharmaceutiquement acceptable de celui-ci, s'opposent, en les empêchant, les limitant, ou en réparant leurs conséquences, aux effets pro-neuropathiques du composé additionnel cytostatique ou anti-mitotique.

### Description des figures

### Figure 1

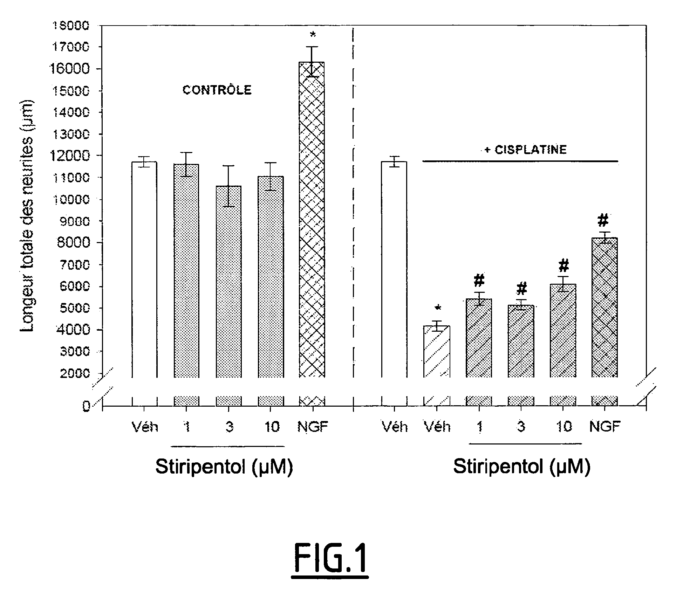

La figure 1 représente les effets du véhicule (témoins), du stiripentol (1, 3, 10 µM) et du NGF (5 ng/ml) sur la longueur totale des neurites (axe des ordonnées, en µm) en absence et en présence de cisplatine (4,5 µg/ml) durant 24 heures.

Chaque colonne représente la moyenne ± écart standard à la moyenne (ESM, n=12 mesures par groupe).

Le symbole étoile (*) représente p<0,05 comparés aux témoins respectifs (Véhicule ; sans cisplatine) ; le symbole dièse (#) représente p<0,05 comparés aux contrôles traités avec le cisplatine (ANOVA ou épreuve de Kruskall Wallis suivie du test de Student Newmann Keuls ou de Dunn respectivement).

### Figure 2

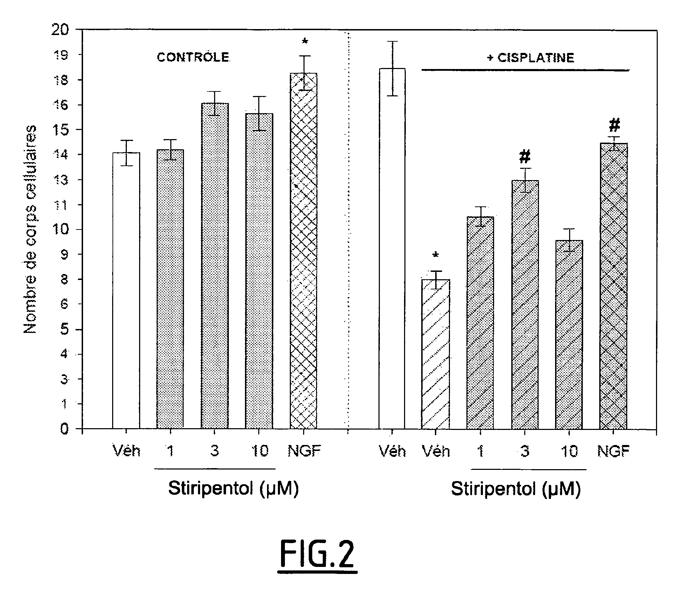

La figure 2 représente les effets du véhicule (témoins), du stiripentol (1, 3, 10 µM) et du NGF (5 ng/ml) sur le nombre de corps cellulaires (axe des ordonnées) en absence et en présence de cisplatine (4,5 µg/ml) durant 24 heures.

Chaque colonne représente la moyenne ± ESM (n=12 mesures par groupe).

Le symbole étoile (*) représente p<0,05 comparés aux témoins respectifs (Véhicule ; sans cisplatine) ; le symbole dièse (#) représente p<0,05 comparés aux contrôles traités avec le cisplatine (épreuve de Kruskall Wallis suivie du test de Dunn).

### Figure 3

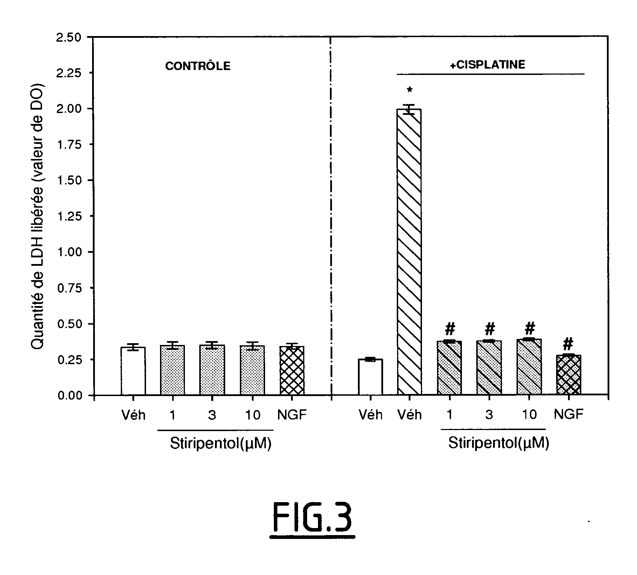

La figure 3 représente les effets du véhicule (témoins), du stiripentol (1, 3, 10 µM) et du NGF (5 ng/ml) sur la libération cellulaire de LDH (axe des ordonnées, valeur de densité optique (DO)) en absence ou en présence de cisplatine (4,5 µg/ml) durant 24 heures.

Chaque colonne représente la moyenne ± ESM (n=6 mesures/groupe).

Le symbole étoile (*) représente p<0,05 comparés aux témoins respectifs (Véhicule ; sans cisplatine) ; le symbole dièse (#) représente p<0,05 comparés aux contrôles traités avec le cisplatine (épreuve de Kruskall Wallis suivie du test de Dunn).

### Exemple

Le principe de l'étude réalisée par les inventeurs était d'étudier les effets neuroprotecteurs du stiripentol dans un modèle *in vitro* de neuropathies périphériques. Plus précisément, ils ont étudié la survie de neurones sensitifs extraits de ganglions rachidiens dorsaux d'embryons de rat mis en culture en présence d'un agent cytotoxique, le cisplatine, co-appliqué avec du stiripentol.

### Méthodes

Une co-culture durant 5 jours de neurones sensitifs (∼5-10% de la population cellulaire cultivée) associés à des cellules de Schwann et des fibroblastes (∼95-90% de la population cellulaire cultivée) est réalisée dans un milieu approprié comme cela est décrit dans Hall et al. (1997) J.Neurosci. 17:2775-2784.

Au 5ème jour, la culture cellulaire est mise à incuber durant 24 heures en présence de DMSO (0,1%, liquide véhicule) et de stiripentol (lot 162, Biocodex, France) aux concentrations de 1, 3, et 10 µM ou de NGF (réf 13290-010 Invitrogen, France) (5 ng/ml), choisi comme produit de référence. Ces différentes substances sont associées ou non au cisplatine (réf P4394, Sigma, France) (4,5 µg/ml).

On détermine alors la densité des réseaux neuritiques (axones et dendrites) marquée par un anticorps anti-β-tubuline (réf T8660, Sigma, France) en mesurant leur longueur, ainsi que le nombre de corps cellulaires objectivés à l'aide d'un anticorps anti-MAP2 (réf M4403, Sigma, France) comme cela est indiqué par Gill & Windebank (1998) J. Clin. Invest. 101:2842-2850. Par ailleurs, on détermine la quantité de lactate déshydrogènase (LDH) (Kit de détection, réf 1 644 793, Roche) libérée dans le milieu extracellulaire, dont la quantité est proportionnelle au nombre de cellules endommagées ou mortes du fait de la lyse cellulaire induite par le cisplatine, comme cela est indiqué par Koh & Choi (1987) J Neurosci Methods 20:83-90.

### Résultats

Comme attendu, le cisplatine induit une diminution de la densité des réseaux neuritiques (**Fig. 1**) ainsi qu'une dégénérescence des corps cellulaires (**Fig. 2**) associée à une lyse cellulaire **(****Fig. 3****)***. A contrario,* le NGF à la concentration de 5 ng/ml présente une activité neurotrophique (**Fig. 1****,** **Fig. 2**) et neuroprotectrice (**Fig. 1 à 3**).

S'agissant du stiripentol, il a pour effet de réduire la diminution de la densité des réseaux neuritiques (**Fig. 1**) et du nombre de corps cellulaires neuronaux (**Fig. 2**) induite par le cisplatine. En outre, les résultats obtenus après dosage de LDH révèlent un effet neuroprotecteur du stiripentol (**Fig. 3**). Ainsi, dans ce modèle *in vitro* de neuropathie périphérique, le stiripentol (1-10 µM) présente une activité neuroprotectrice de même ordre que celle du NGF (5 ng/ml).

## Revendications

1. Composé de formule (I) suivante : 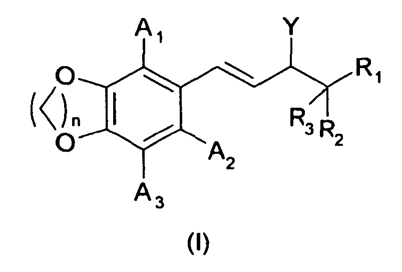 dans laquelle :
- n représente 1 ou 2,
- A₁, A₂ et A₃, identiques ou différents, représentent un atome d'hydrogène, un atome d'halogène ou un groupe alkyle linéaire ou ramifié de 1 à 4 atomes de carbone,
- R₁, R₂ et R₃ représentent indépendamment un atome d'hydrogène ou un groupe alkyle linéaire ou ramifié de 1 à 4 atomes de carbones, et
- Y représente -OH, =O ou -SH ;
ou un sel pharmaceutiquement acceptable de celui-ci,
pour son utilisation dans la prévention ou le traitement des neuropathies périphériques.

2. Composé ou sel pharmaceutiquement acceptable de celui-ci pour son utilisation selon la revendication 1, de formule (II) suivante : 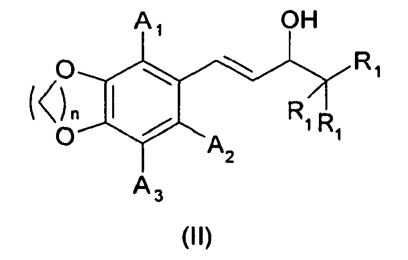 dans laquelle n, A₁, A₂, A₃ et R₁ sont tels que définis dans la revendication 1.

3. Composé ou sel pharmaceutiquement acceptable de celui-ci pour son utilisation selon la revendication 1 ou 2, de formule (III) suivante :

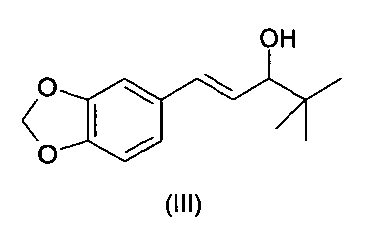

4. Composé ou sel pharmaceutiquement acceptable de celui-ci pour son utilisation selon l'une des revendications 1 à 3, dans lequel la neuropathie périphérique est associée à une douleur.

5. Composé ou sel pharmaceutiquement acceptable de celui-ci pour son utilisation selon l'une des revendications 1 à 4, dans lequel la neuropathie périphérique est liée à la prise d'un médicament.

6. Composé ou sel pharmaceutiquement acceptable de celui-ci pour son utilisation selon la revendication 5, dans lequel le médicament est cytostatique ou anti-mitotique.

7. Composé ou sel pharmaceutiquement acceptable de celui-ci pour son utilisation selon l'une des revendications 1 à 6, pour une administration à une dose unitaire de 5 mg/kg à 100 mg/kg.

8. Composé ou sel pharmaceutiquement acceptable de celui-ci pour son utilisation selon l'une des revendications 1 à 7, pour une administration avec un régime de dosage de 10 mg/kg/j à 200 mg/kg/j.

9. Composé ou sel pharmaceutiquement acceptable de celui-ci pour son utilisation selon l'une des revendications 1 à 8, sous une forme convenant pour une administration par voie orale ou rectale.

10. Composé ou sel pharmaceutiquement acceptable de celui-ci pour son utilisation selon l'une des revendications 1 à 9, sous forme d'une poudre, de sachets, de comprimés, de gélules ou de suppositoires.

11. Composé ou sel pharmaceutiquement acceptable de celui-ci pour son utilisation selon l'une des revendications 1 à 10, combiné à au moins un composé additionnel destiné à la prévention ou au traitement des neuropathies périphériques.

12. Composé ou sel pharmaceutiquement acceptable de celui-ci pour son utilisation selon la revendication 11, dans lequel le composé additionnel destiné à la prévention ou au traitement des neuropathies périphériques est sélectionné dans le groupe constitué du NGF, de BDNF, de CNTF, d'IGF-I, de NT-3 et de la L-carnitine.

13. Composé ou sel pharmaceutiquement acceptable de celui-ci pour son utilisation selon l'une des revendications 1 à 12, combiné à au moins un composé additionnel cytostatique ou anti-mitotique.

14. Composé ou sel pharmaceutiquement acceptable de celui-ci pour son utilisation selon la revendication 13, dans lequel le composé additionnel cytostatique ou anti-mitotique est sélectionné dans le groupe constitué du cisplatine, du satraplatine, du carboplatine, de l'oxaliplatine, de la vincristine, de la vinblastine, de la doxorubicine, et des taxoïdes.

15. Composition pharmaceutique, comprenant à titre de substances actives, au moins un composé de formule (I) tel que défini dans l'une des revendications 1 à 3, ou un sel pharmaceutiquement acceptable de celui-ci, et au moins un composé additionnel tel que défini dans la revendication 12 et/ou au moins un composé additionnel tel que défini dans la revendication 13 ou 14, éventuellement en association avec un véhicule pharmaceutiquement acceptable.

16. Produits contenant :
- au moins un composé de formule (I) tel que défini dans l'une des revendications 1 à 3, ou un sel pharmaceutiquement acceptable de celui-ci, et
- au moins un composé additionnel tel que défini dans la revendication 11 ou 12, et/ou
- au moins un composé additionnel tel que défini dans la revendication 13 ou 14, comme produit de combinaison pour une utilisation simultanée, séparée ou étalée dans le temps pour la prévention ou le traitement des neuropathies périphériques.

## Claims

1. A compound of the following formula (I): 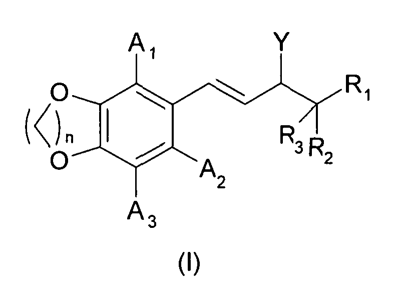 wherein:
- n represents 1 or 2,
- A₁, A₂ and A₃, which may be identical or different, represent a hydrogen atom, a halogen atom or a linear or branched alkyl group having from 1 to 4 carbon atoms,
- R₁, R₂ and R₃ represent independently a hydrogen atom or a linear or branched alkyl group having from 1 to 4 carbon atoms, and
- Y represents -OH, =O or -SH ;
or a pharmaceutically acceptable salt thereof,
for use in the prevention or treatment of peripheral neuropathies.

2. The compound or pharmaceutically acceptable salt thereof for use according to claim 1, wherein the compound or pharmaceutically acceptable salt thereof is of the following formula (II): 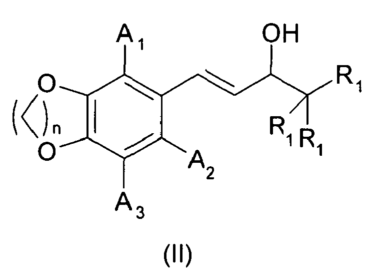 wherein n, A₁, A₂, A₃ and R₁ are as defined in claim 1.

3. The compound or pharmaceutically acceptable salt thereof for use according to claim 1 or 2, wherein the compound or pharmaceutically acceptable salt thereof is of the following formula (III):

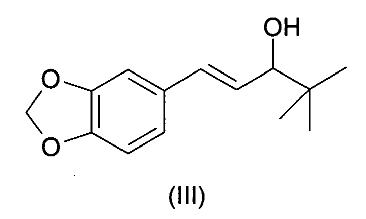

4. The compound or pharmaceutically acceptable salt thereof for use according to any of claims 1 to 3, wherein the peripheral neuropathy is associated with pain.

5. The compound or pharmaceutically acceptable salt thereof for use according to any of claims 1 to 4, wherein the peripheral neuropathy is associated with the taking of a medicament.

6. The compound or pharmaceutically acceptable salt thereof for use according to claim 5, wherein the medicament is cytostatic or antimitotic.

7. The compound or pharmaceutically acceptable salt thereof for use according to any of claims 1 to 6, wherein the compound or pharmaceutically acceptable salt thereof is administered at a unit dose of from 5 mg/kg to 100 mg/kg.

8. The compound or pharmaceutically acceptable salt thereof for use according to any of claim 1 to 7, wherein the compound or pharmaceutically acceptable salt thereof is administered at a dose regimen of from 10 mg/kg/d to 200 mg/kg/d.

9. The compound or pharmaceutically acceptable salt thereof for use according to any of claims 1 to 8, wherein the compound or pharmaceutically acceptable salt thereof is in a form suitable for administration by the oral or rectal route.

10. The compound or pharmaceutically acceptable salt thereof for use according to any of claims 1 to 9, wherein the compound or pharmaceutically acceptable salt thereof is in the form of a powder, sachets, tablets, capsules or suppositories.

11. The compound or pharmaceutically acceptable salt thereof for use according to any of claims 1 to 10, wherein the compound or pharmaceutically acceptable salt thereof is combined with at least one additional compound intended for the prevention or treatment of peripheral neuropathies.

12. The compound or pharmaceutically acceptable salt thereof for use according to claim 11, wherein the additional compound intended for the prevention or treatment of peripheral neuropathies is selected from the group consisting of NGF, BDNF, CNTF, IGF-I, NT-3 and L-carnitine.

13. The compound or pharmaceutically acceptable salt thereof for use according to any of claims 1 to 12, wherein the compound or pharmaceutically acceptable salt thereof is combined with at least one additional cytostatic or antimitotic compound.

14. The compound or pharmaceutically acceptable salt thereof for use according to claim 13, wherein the additional cytostatic or antimitotic compound is selected from the group consisting of cisplatin, satraplatin, carboplatin, oxaliplatin, vincristine, vinblastine, doxorubicin, and taxoids.

15. A pharmaceutical composition, comprising as active substances at least one compound of formula (I) as defined in any of claims 1 to 3, or a pharmaceutically acceptable salt thereof, and at least one additional compound as defined in claim 12 and/or at least one additional compound as defined in claim 13 or 14, optionally in association with a pharmaceutically acceptable vehicle.

16. Products containing:
- at least one compound of formula (I) as defined in any of claims 1 to 3, or a pharmaceutically acceptable salt thereof, and
- at least one additional compound as defined in claim 11 or 12, and/or
- at least one additional compound as defined in claim 13 or 14
as a combined preparation for simultaneous, separate or sequential use in the prevention or treatment of peripheral neuropathies.

## Patentansprüche

1. Verbindung der folgenden Formel (I): 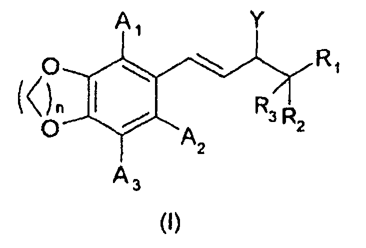 in der:
- n 1 oder 2 darstellt,
- A₁, A₂ und A₃, die gleich oder unterschiedlich sind, ein Wasserstoffatom, ein Halogenatom oder eine lineare oder verzweigte Alkylgruppe mit 1 bis 4 Kohlenstoffatomen darstellen,
- R₁, R₂ und R₃ unabhängig ein Wasserstoffatom oder eine lineare oder verzweigte Alkylgruppe mit 1 bis 4 Kohlenstoffatomen darstellen und
- Y -OH, =O oder -SH darstellt;
oder ein pharmazeutisch verträgliches Salz derselben zur Verwendung bei der Prävention oder Behandlung peripherer Neuropathien.

2. Verbindung der folgenden Formel (II): 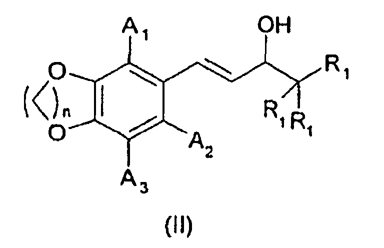 in der n, A₁, A₂, A₃ und R₁ wie in Anspruch 1 definiert sind, oder pharmazeutisch verträgliches Salz davon zur Verwendung gemäß Anspruch 1.

3. Verbindung der folgenden Formel (III): 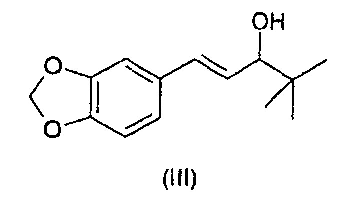 oder ein pharmazeutisch verträgliches Salz davon zur Verwendung gemäß Anspruch 1 oder 2.

4. Verbindung oder pharmazeutisch verträgliches Salz davon zur Verwendung gemäß einem der Ansprüche 1 bis 3, wobei die periphere Neuropathie mit Schmerz assoziiert ist.

5. Verbindung oder pharmazeutisch verträgliches Salz derselben zur Verwendung gemäß einem der Ansprüche 1 bis 4, wobei die periphere Neuropathie durch Einnahme eines Medikaments bedingt ist.

6. Verbindung oder pharmazeutisch verträgliches Salz davon zur Verwendung gemäß Anspruch 5, wobei das Medikament ein Zytostatikum oder Antimitotikum ist.

7. Verbindung oder pharmazeutisch verträgliches Salz davon zur Verwendung gemäß einem der Ansprüche 1 bis 6 für eine Verabreichung in einer Einheitsdosis mit 5 mg/kg bis 100 mg/kg.

8. Verbindung oder pharmazeutisch verträgliches Salz davon zur Verwendung gemäß einem der Ansprüche 1 bis 7 für eine Verabreichung mit einem Dosierungsplan von 10 mg/kg/Tag bis 200 mg/kg/Tag.

9. Verbindung oder pharmazeutisch verträgliches Salz davon zur Verwendung gemäß einem der Ansprüche 1 bis 8 in einer Form, die für eine Verabreichung auf oralem oder rektalem Weg zweckmäßig ist.

10. Verbindung oder pharmazeutisch verträgliches Salz davon zur Verwendung gemäß einem der Ansprüche 1 bis 9 in der Form eines Pulvers, von Beuteln, Tabletten, Gelatinekapseln oder Suppositorien.

11. Verbindung oder pharmazeutisch verträgliches Salz davon zur Verwendung gemäß einem der Ansprüche 1 bis 10, kombiniert mit wenigstens einer zusätzlichen Verbindung, die zur Prävention oder Behandlung von peripheren Neuropathien bestimmt ist.

12. Verbindung oder pharmazeutisch verträgliches Salz davon zur Verwendung gemäß Anspruch 11, wobei die zusätzliche Verbindung, die zur Prävention oder Behandlung von peripheren Neuropathien bestimmt ist, ausgewählt ist aus der Gruppe, bestehend aus NGF, BDNF, CNTF, IGF-I, NT-3 und L-Carnitin.

13. Verbindung oder pharmazeutisch verträgliches Salz davon zur Verwendung gemäß einem der Ansprüche 1 bis 12 kombiniert mit wenigstens einer zusätzlichen zytostatischen oder antimitotischen Verbindung.

14. Verbindung oder pharmazeutisch verträgliches Salz davon zur Verwendung gemäß Anspruch 13, wobei die zusätzliche zytostatische oder antimitotische Verbindung ausgewählt ist aus der Gruppe, bestehend aus Cisplatin, Satraplatin, Carboplatin, Oxaliplatin, Vincristin, Vinblastin, Doxorubicin und Taxoiden.

15. Pharmazeutische Zusammensetzung, umfassend als aktive Substanzen wenigstens eine Verbindung der Formel (I), wie sie in einem der Ansprüche 1 bis 3 definiert ist, oder ein pharmazeutisch verträgliches Salz davon und wenigstens eine zusätzliche Verbindung, wie sie in Anspruch 12 definiert ist, und/oder wenigstens eine zusätzliche Verbindung, wie sie in Anspruch 13 oder 14 definiert ist, gegebenenfalls in Verbindung mit einem pharmazeutisch verträglichen Vehikel.

16. Produkte, enthaltend:
- wenigstens eine Verbindung der Formel (I), wie sie in einem der Ansprüche 1 bis 3 definiert ist, oder ein pharmazeutisch verträgliches Salz davon und
- wenigstens eine zusätzliche Verbindung, wie sie in Anspruch 11 oder 12 definiert ist, und/oder
- wenigstens eine zusätzliche Verbindung, wie sie in Anspruch 13 oder 14 definiert ist, als Kombinationsprodukt zur gleichzeitigen, getrennten oder über die Zeit verteilten Verwendung zur Prävention oder Behandlung von peripheren Neuropathien.
